# NEW EUROPEAN PATENT SPECIFICATION

(11) **EP 2 651 323 B2**
(45) Date of publication and mention of the opposition decision: **07.08.2019**
(45) Mention of the grant of the patent: 27.07.2016
(21) Application number: 10798009.6
(22) Date of filing: 17.12.2010
(51) Int. Cl.: A61B 17/88, A61B 17/80

(54) **SURGICAL DEVICE FOR BENDING AN IMPLANT AND IMPLANT SYSTEM COMPRISING THE DEVICE**
CHIRURGISCHE VORRICHTUNG ZUM BIEGEN EINES IMPLANTATS SOWIE IMPLANTATSYSTEM MIT DER VORRICHTUNG
DISPOSITIF CHIRURGICAL POUR COURBER UN IMPLANT ET SYSTÈME D'IMPLANT COMPRENANT LE DISPOSITIF

(43) Date of publication of application: 23.10.2013
(73) Proprietor: Stryker European Holdings I, LLC, Kalamazoo, MI 49002 (US)
(72) Inventor: KNOEPFLE, Christian, 78166 Donaueschingen (DE)
(74) Representative: Röthinger, Rainer
(86) International application number: PCT/EP2010/007749
(87) International publication number: WO 2012/079611

(56) References cited:
- DE-A1- 3 724 479
- DE-U1- 29 510 041
- GB-A- 2 448 123
- US-A- 5 373 860
- US-A- 5 413 577
- US-A1- 2002 159 344
- KURT LANGE: "Umformtechnik, Handbuch für Industrie und Wissenschaft", , vol. 3, 1990, XP055365610, ISBN: 978-3-662-10687-7
- OEHLER ET AL: "tanz- und Ziehwerkzeuge", , XP055365617, ISBN: 978-3-642-53110-1
- SCHÄFFLER KG: "Technisches Taschenbuch", , 7 May 2002 (2002-05-07), pages 108-111, XP055365621,

## Description

### Technical Field

The present disclosure generally relates to surgical devices and implants. In particular, a surgical device for bending an implant that is to be applied to the cranio-facial region is described.

### Background

Fractures in the cranio-facial region such as the orbita are conventionally treated using a large variety of implants such as meshes, plates or combinations thereof. In the past such implants have been delivered in a flat shape. A surgeon was then obliged to intraoperatively bend the implant to reproduce the anatomical features of the cranio-facial region to be treated. For this reason the fitting accuracy of the implant strongly depended on the experience of the individual surgeon.

In an effort to provide an adequate fitting accuracy regardless of the skills of the individual surgeon it was proposed to distribute cranio-facial implants in a pre-formed state. As an example, US 7,662,155 B2 teaches a pre-formed implant for use as a replacement for an orbita bottom. The implant comprises multiple segments, with a first segment being formed in accordance with the orbita bottom and comprising an elevation, a second segment being formed according to the medial orbita wall, and a third segment being arranged for fixing the implant at a lateral orbita edge.

It has been found that implants distributed in a pre-formed state are more susceptible to being damaged prior to implantation than flat implants. Since the implants are often manufactured from an essentially plastically deformable material such as metal, the pre-formed implants may easily get deformed upon removal from their transport packaging, in the course of sterilization (if needed) or when being cut to a desired shape by a surgeon. Pre-formed implants that have been unintentionally deformed prior to implantation are either thrown away or have to be tediously re-bent by the surgeon.

DE 295 10 041 U1 discloses a device for bending a mesh. The device is formed as a plier and includes a first mould member and a second mould member which are adapted to partially receive the mesh between them. The first mould member has a convex surface and the second mould member has a concave surface corresponding in shape to the convex surface of the first mould member. The device further includes two arms coupled via a joint. Once the two arms are pressed together, they convert a manually exerted force into an actuation force acting on the first and second mould members to bend the mesh received between the two mold members.

Further technological background can be found in US 5,373,860 A.

### Summary

Accordingly, there is a need to overcome the problems associated with unintentional deformations of pre-formed implants by providing a surgical device facilitating an intraoperative bending of an implant for the cranio-facial region.

To this end, an implant system comprising a surgical device for bending an implant and the implant that has been bent is provided, which comprises first and second mould members adapted to at least partially receive the implant between them, wherein the mould members are shaped in three dimensions in ac-cordance with one or more cranio-facial features, and an actuation mechanism adapted to convert a manually exerted force into an actuation force acting on at least one of the mould members to bend the implant received between the mould members.

One of the mould members may essentially be concavely shaped and the other of the mould members may essentially be convexly shaped. Moreover, one of the mould members may define a positive mould and the other of the mould members may define a negative mould. The mould members are shaped in accordance with at least one portion of the orbita, such as the orbita bottom. Additionally, or in the alternative, the mould members may be shaped in accordance with a medial orbital wall adjacent to the orbita bottom.

The mould members are over-shaped relative to the bent implant to take into account a partial elastic recovery of the implant after bending so as to ensure that the shape of the elastically recovered implant exactly conforms to the one or more cranio-facial features. As an example, the mould members may be over-bent to ensure that the shape of the elastically recovered implant exactly conforms to the one or more anatomical features in question.

According to a first variant, the mould members may fixedly be mounted to the surgical device. According to a second variant, the mould members may removably (i.e., detachably) be mounted to the surgical device. The second variant is particularly useful in case the surgical device is used in combination with mould members having a patient-specific shape as the actuation mechanism may be re-used for multiple patients. The second variant is, however, also useful when the mould member are shaped in accordance with a generic model of the cranio-facial region as in this case sets of mould members shaped in accordance with different generic models (e.g., for patients of different genders or different ages) may be used for different patients in combination with a single actuation mechanism.

The surgical device comprises a fixation mechanism for the implant. The fixation mechanism is configured to prevent an unintentional movement of the implant received between the two mould members prior to or during the bending operation. As such, the fixation mechanism defines a fixed relative position between the implant and at least one of the mould members for the bending operation.

The fixation mechanism comprises two or more protrusions provided on a face of at least one of the mould members. The two or more protrusions are adapted to be inserted into one or more corresponding openings of the implant. Such implant openings are adapted to receive bone screws. In one implementation, the two or more protrusions may be configured to cooperate with the one or more corresponding openings of the implant in a form-fitting manner.

The fixation mechanism may additionally comprise one or more abutment elements provided on a face of at least one of the mould members. The one or more abutment elements may be adapted to be brought into abutment with one or more portions of the implant for fixation purposes. The abutment may occur in at least one of a perimetral and an inner region of the implant.

The first and second mould members may be coupled to each other. As an example, the first and second mould members may be pivotably coupled to each other.

The surgical device may be configured in various forms. In a pliers configuration, the surgical device may comprise two pivotably connected handles coupled to the first and second mould members, respectively. The handles may be located on an opposite side of the first and second mould members with respect to the pivotal coupling and may belong to the actuation mechanism.

In a configuration as a handheld unit, the surgical device may comprise a dedicated force exertion region associated with each of the first and second mould members. The force exertion regions are located on the same side as the mould members with respect to the pivotal coupling (e.g., in a nutcracker-type manner). The actuation mechanism may in this case comprise the force exertion regions. The force exertion regions may be realized in the form handles or surfaces for manually applying pressure.

In a tabletop unit configuration, the surgical device may comprise a base member supporting the first mould member and a handle member coupled (e.g., via a lever arrangement) to the second mould member. In such a configuration the actuation mechanism may comprise the handle member of the tabletop unit.

The implant is adapted to be implanted in the orbita region and, prior to being bent, may have a generally flat shape. Alternatively, the implant may be already be pre-formed (either by the surgeon or upon manufacturing) prior to the bending operation. As an example, the implant may be an orbita implant intended for being implanted in the region of an orbita bottom and/or in the region of a medial orbita wall.

The implant may be formed from a bio-compatible material having at least partially plastic properties for being bent by the surgical device. In one exemplary realization, the implant is made from metal such as titanium or stainless steel. Moreover, the implant may generally have a thickness which permits bending the implant between the two mould members by the surgeon or other surgical personnel. For example, the implant may have a thickness between 0.1 mm and 3.5 mm depending on the region of implantation. As an example, an orbita implant may have a thickness between 0.2 mm to 1.0 mm.

The implant may generally be realized in the form of a mesh or a plate. The implant may also be realized as comprising a mesh structure and one or more further structures. The further structures may comprise stiffening portions defining, for example, an outer periphery of the implant. Such a realization of the implant is sometimes also referred to as "plate". The implant comprises two or more openings adapted to receive bone screws for attaching the implant to bone. The openings may generally be arranged in a peripheral portion of the implant. In an exemplary realization as orbita implant, the openings may, for example, be arranged for fixing the implant at an orbita edge (either within or outside the orbita).

The implant system may comprise a single surgical device or multiple surgical devices. In the latter case, the implant system may comprise a first surgical device having a first pair of mould members shaped in accordance with a first anatomical side and a second device having a second pair of mould members shaped in accordance with a second anatomical side opposite the first anatomical side. In another implementation, the surgical device may be configured to removably receive the mould members, wherein a first pair of mould members is shaped in accordance with a first anatomical side and a second pair of mould members is shaped in accordance with a second anatomical side opposite to the first anatomical side. Still further, the implant system may comprise a first implant shaped in accordance with a first anatomical side and a second implant shaped in accordance with a second anatomical side opposite to the first anatomical side. The first anatomical side and the second anatomical side may be opposite sides (i.e., the right side and the left side) of the cranium or the face, respectively.

Also provided is a method for bending an implant using the above implant system. Said method comprises the steps defined in enclosed claim 19.

### Brief Description of the Drawings

Further aspects, realizations and advantages of the surgical device and the implant system presented herein will now be described with reference to exemplary embodiments illustrated in the drawings, wherein
- Figs. 1A and 1B: show a top view and a side view of a first embodiment of a surgical device in the form of moulding pliers;
- Figs. 2A and 2B: show perspective views of mould member embodiments in the form of a negative mould and a positive mould, respectively;
- Fig. 3: shows a perspective view of the relative location of the two mould members of Figs. 2A and 2B prior to reception of an implant;
- Fig. 4: shows a perspective view of the relative location of the two mould members of Figs. 2A and 2B after reception of an exemplary orbita implant;
- Fig. 5: shows a perspective view of a second embodiment of a surgical device in the form of a tabletop unit; and
- Fig. 6: shows a perspective view of a third embodiment of a surgical device in the form of a foldable handheld unit.

### Detailed Description

In the following embodiments, for purposes of explanation and not limitation, specific details of various exemplary surgical devices are described.

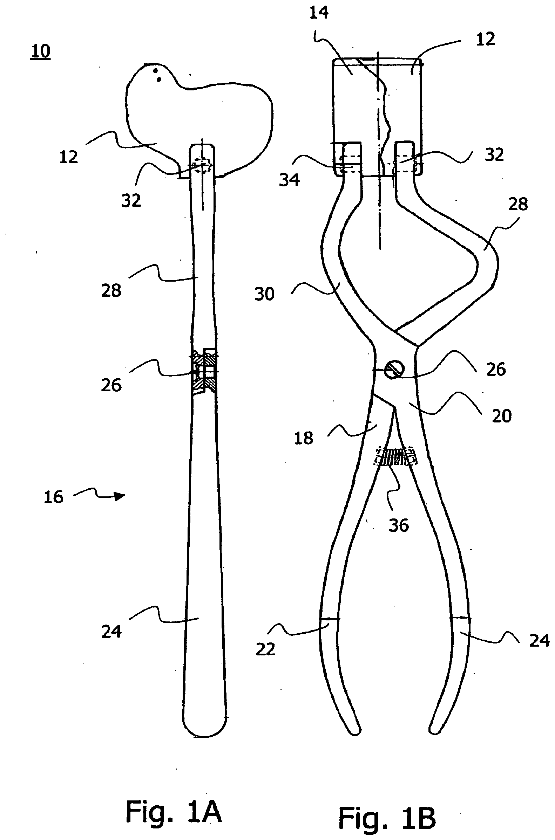

Figs. 1A and 1B illustrate a moulding pliers embodiment of a surgical device 10 for bending an orbita implant. The surgical device 10 comprises two mould members 12, 14 adapted to at least partially receive the implant (not shown in Figs. 1A and 1B) between them. The surgical device 10 further comprises an actuation mechanism 16 adapted to press the two mould members 12, 14 against each other to bend the implant received between them. In the embodiment illustrated in Figs. 1A and 1B, the actuation mechanism 16 comprises a pair of pliers 18, 20. The pliers 18, 20 include handles 22, 24 secured together by a pivot pin 26.

The handles 22, 24 extend beyond the pivot pin 26 as jaws 28, 30. As may be seen from Figs. 1A and 1B, each of the mould members 12, 14 is detachably mounted to one of the jaws 28, 30 by a removable securing bolt 32, 34. The mould members 12, 14 can thus be exchanged by other mould members as needed, while the pliers 18, 20 can be re-used.

As illustrated in Fig. 1B, the surgical device further comprises a spring member 36 located between the pliers 18, 20 in the region of the handles 22, 24 and urging the handles 22, 24 away from each other. In the non-actuated state of the surgical device (not shown), the handles 22, 24 are thus located at a maximum distance from each other. For this reason the two mould members 12, 14 at the opposite side of the pivot pin 26 relative to the handles 22, 24 are also spaced at a maximum distance from each other in the non-actuated state, so that the orbita implant can be inserted between them as will be described in more detail below.

When actuating the handles 22, 24 (i.e., when manually applying a force on the handles 22, 24), the spring member 36 is compressed and the handles 22, 24 approach each other until the two mould members 12, 14 come into abutment as illustrated in Fig. 1B (which shows the actuated state of the surgical device 10). When the mould members 12, 14 are in the process of coming into abutment, the implant received between them is bent in accordance with the shape of the mould members 12, 14 as the pliers 18, 20 convert the manually exerted force on the handles 22, 24 into an actuation force that presses the two mould members 12, 14 against each other.

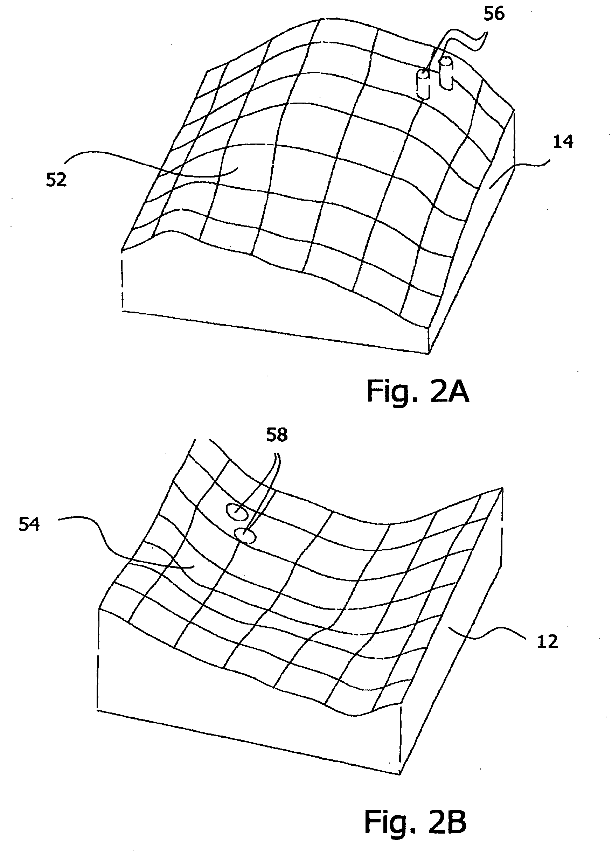

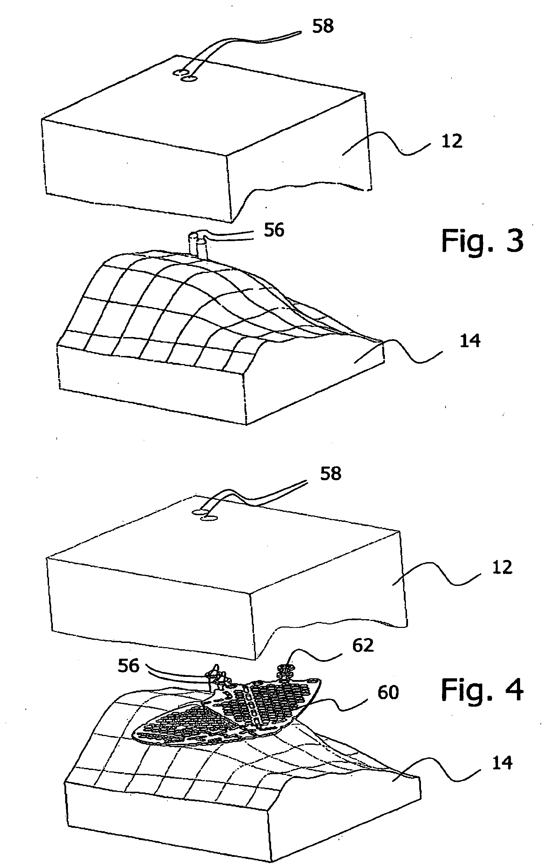

In the following, the configuration of the two mould members 12, 14 will be described in more detail with reference to Figs. 2A, 2B, 3 and 4. Fig. 2A shows a perspective view of mould member 14, Fig. 2B shows a perspective view of the mould member 12, Fig. 3 shows a perspective view of the relative positions of the two mould members 12, 14 in the non-actuated state of the surgical device 10, and Fig. 4 shows a perspective view of the two mould members 12, 14 in the non-actuated state with a bone plate 60 received between them.

As becomes apparent from Figs. 2A and 2B, each of the two mould members 12, 14 is three dimensionally shaped in accordance with anatomical features of the orbita bottom and the medial orbita wall. In this regard, mould member 14 of Fig. 2A is essentially convexly shaped and defines the negative mould, whereas mould member 12 of Fig. 2B is essentially concavely shaped and defines the positive mould. When pressing the face 52 of the mould member 14 against the correspondingly shaped face 54 of the mould member 12, the orbita implant 60 arranged between them will thus be bent in accordance with the anatomical shape of the orbita floor and the medial orbita wall as defined by the three dimensional shape of the mould member faces 52, 54 (see Fig. 4).

The faces 52, 54 of the mould members 12, 14 are shaped in accordance with a generic model of the orbita bottom and the medial orbita wall. This generic model has been derived by suitably averaging anatomical data of a plurality of individuals. The anatomical data can be derived by Computer Tomography (CT) or any other anatomical imaging method known in the art. Different sets of mould members 12, 14 may be derived from different generic models (e.g., generic models for female individuals, for male individuals, for children, for adults, and so on). As the mould members 12, 14 are removably mounted on the surgical device 10, it is possible to exchange the mould members 12, 14 depending on the patient to be treated (and, optionally, the implant 60 to be bent). It would, however, also be possible to provide a set of surgical devices 10, with each surgical device 10 having a pair of mould members 12, 14 shaped in accordance with a specific generic model.

In an alternative implementation, the faces 52, 54 of the mould members 12, 14 have a patient-specific shape. In other words, the mould members 12, 14 can be individually shaped based on anatomical data of the particular patient to which the implant 60 is to be applied. The removable mounting of the mould members 12, 14 facilitates a re-use of the actuation mechanism 16 for different patients by simply exchanging the mould members 12, 14.

It should be noted that the faces 52, 54 of the mould members 12, 14 are shaped taking into account a partial elastic recovery of the implant 60 after bending. For this reason, the individual radii defining the curvatures of the three dimensionally shaped faces 52, 54 are selected to be slightly smaller than the radii of the corresponding anatomical features of the orbita. In this way it is ensured that after the partial recovery of the implant 60 (following the bending operation), the resulting implant 60 is exactly bent in accordance with the anatomical features of the orbita which are to be reconstructed or treated otherwise.

As shown in Fig. 2A, the face 52 of the mould member 14 comprises two substantially cylindrical protrusions 56 that belong to a fixation mechanism that prevents an unintentional movement of the implant prior to or during the bending operation. The other mould member 12 shown in Fig. 2B comprises two associated holes 58 that are sized to accommodate the protrusions 56 when the two mould members 12, 14 are pressed against each other. The protrusions 56 are intended for being inserted into implant openings 62 as will be described in more detail with reference to Fig. 4 below.

It should be noted that in addition to the protrusions 56, the fixation mechanism could also comprise abutment elements provided, for example, on the face 52 of the mould member 14 shown in Fig. 2A (corresponding structures, such as grooves, for accommodating the abutment elements when the two mould members 12, 14 are pressed together may be provided in the face 54 of the mould member 12). The abutment elements may fully or in sections enclose the outer periphery of the implant 60 so as to prevent any movement thereof when being received between the mould members 12, 14 prior to or during the bending operation.

Fig. 3 illustrates the relative location of the mould members 12, 14 in the non-actuated state of the surgical device 10. In this non-actuated state, the spacing between the two mould members 12, 14 permits to insert the implant 60 between them as illustrated in Fig. 4.

As shown in Fig. 4, the implant 60 comprises two radially extending portions with two linearly arranged screw holes 62 each. Two of the screw holes 62 are threaded on the two protrusions 56 of mould member 14 such that the implant 60 is aligned in a fixed position relative to the three dimensionally shaped face 52 of the mould member 14. An unintentional movement of the implant 60 relative to the face 52 prior to or during the bending operation (and any defective deformation of the implant 60 resulting from a mis-alignment) can thus be prevented. It should be noted that in addition to the two protrusions 56, one or more protrusions may be provided that are intended to cooperate with an interior region of the implant 60 (e.g., that are to be inserted into mesh openings or dedicated fixation openings in an interior region of the implant 60).

The implant 60 of Fig. 4 is an orbita implant and may, for example, be used as a replacement for an orbita bottom (and, if desired, also for a medial orbita wall). The implant 60 is manufactured from titanium and has a thickness of approximately 0.25 mm to 0.5 mm (e.g., of 0.35 mm). As becomes apparent from Fig. 4, the implant 60 comprises an inner mesh structure and a peripheral stiffening structure. The linearly arranged screw holes 62 are intended for receiving bone screws that fix the implant 60 to the orbita edge.

The implant 60 illustrated in Fig. 4 is implanted in the orbita on the left anatomical side of a patient. It will be appreciated that an implant system additionally comprising another implant for the opposite right anatomical side could be provided. In this regard, a different pair of mould members will be needed for its bending. In one implementation, the implant system may comprise a first surgical device carrying mould members for the left anatomical side and a second surgical device carrying mould members for the right anatomical side.

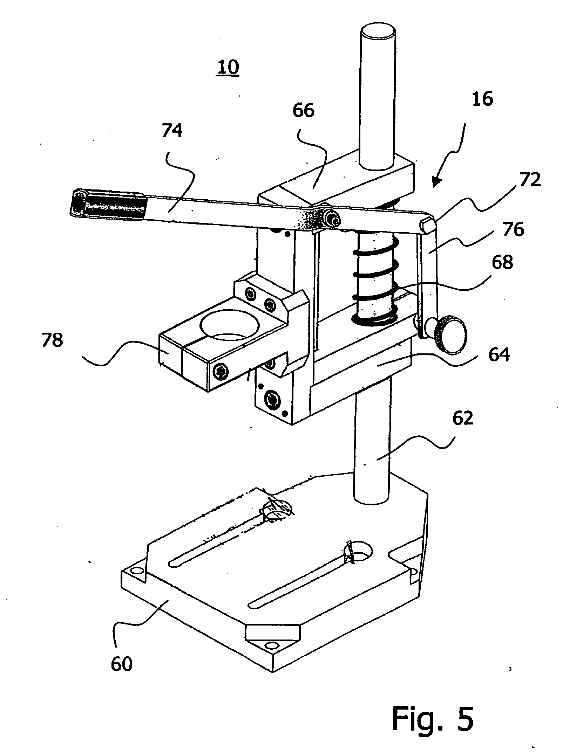

Fig. 5 shows a perspective view of a second embodiment of a surgical device 10 in the form of a tabletop unit. The surgical device 10 illustrated in Fig. 5 may be equipped with the same mould members (illustrated in Figs. 2A, 2B, 3 and 4) as the surgical device illustrated in Figs. 1A and 1B.

The surgical device 10 of Fig. 5 comprises a base member 60 as well as a rod 52 mounted on the base member 60 and extending perpendicularly relative to the base member 60. An abutment plate 64 is fixedly mounted on the rod 52. Additionally, a carriage 66 (belonging to an actuation mechanism 16) is slidingly received on the rod 62. A slightly compressed spring member 68 is arranged between the abutment plate 64 and the carriage 66. The spring member 66 biases the carriage 66, with respect to the abutment plate 64, away from the base member 60 so that in a non-actuated state the carriage 66 comes into abutment with a lower face of the abutment plate 64.

The actuation mechanism 16 further comprises a lever assembly 72 with a handle 74 and a lever member 76. The handle 74 is pivotably coupled to both the carriage 66 and a first end of the lever member 68. A second end of the lever member 68 is pivotably coupled to the abutment plate 64.

As illustrated in Fig. 5, the carriage 66 carries a support member 78 that is configured to receive, via an adapter not shown in Fig. 5, the mould member 12 of Fig. 2B. The mould member 14 of Fig. 2A, on the other hand, is supported on the base member 60. When mounted on the surgical device 10, the two mould members 12, 14 are aligned such that the protrusions 56 will be received in the holes 58 upon bringing the faces 52, 54 of the mould members 12, 14 into abutment.

To bring the faces 52, 54 of the mould members 12, 14 into abutment, and to bend the implant 60 received between them, the lever assembly 72 has to be actuated. To this end, the handle 74 is pressed down towards the base member 60 against the biasing force of the spring member 68. The spring member 68 is compressed and the carriage 66, with the mould member 12, is moved downwardly. The actuation mechanism 16 thus converts a force manually exerted on the handle 74 into an actuation force pressing the two mould members 12, 14 against each other and bending the implant 60 received between them. When the handle 74 is released, the spring member 68 pushes the carriage 66 with the mould member 12 away from the base member 60 and the mould member 14, and the implant 60 can be removed.

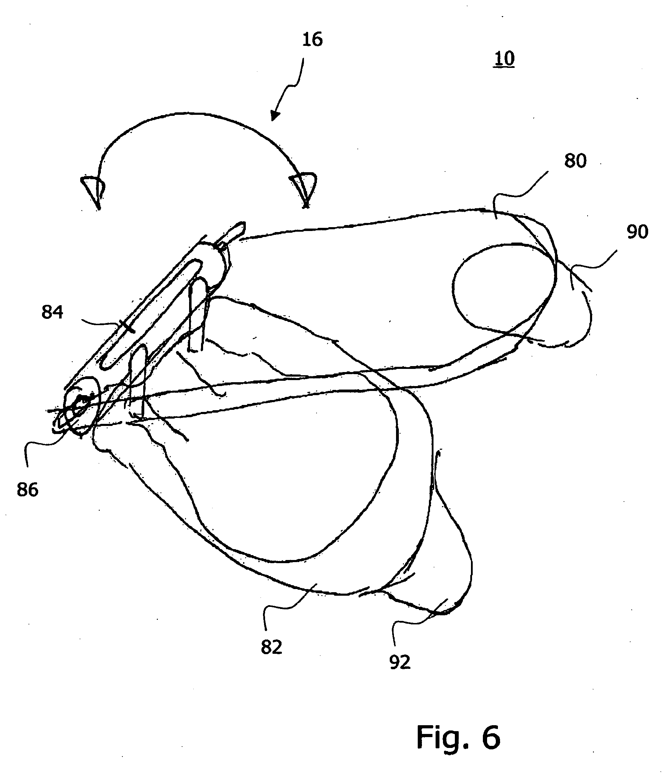

Fig. 6 shows a perspective view of a third embodiment of a surgical device 10 in the form of a foldable handheld unit of the nutcracker-type. The surgical device 10 of Fig. 6 may be equipped with the same or suitably adapted (e.g., thinner) mould members as illustrated in Figs. 2A, 2B, 3 and 4.

The surgical device 10 of Fig. 6 comprises two opposite support members 80, 82 that are laterally connected by a hinge mechanism 84 comprising a pivot pin 86. Each of the support members 80, 82 is configured to receive one mould member similar to the mould members 12, 14 of Figs. 2A, 2B, 3 and 4. In an alternative embodiment, the mould members could directly be attached to each other via the hinge mechanism 84.

Each of the support members 80, 82 is provided with a tab 90, 92 belonging to an actuation mechanism 16 of the surgical device 10. The tabs 90, 92 constitute force exertion regions for manually applying pressure on the two support members 80, 82. As illustrated in Fig. 6, the tabs 90, 92 are located on the same side as the mould members received by the support members 80, 82 with respect to the pivotal pin 86.

For bending an implant such as the implant 60 illustrated in Fig. 4, the surgical device 10 of Fig. 6 is first brought to the non-actuated state as illustrated in Fig. 6. Then, in a next step, the implant is located between the faces of the mould members of the surgical device 10. Then, the surgical device 10 is actuated in accordance with a folding movement by manually exerting a pressing force on the taps 90, 92 so as to bring the faces of the mould members into abutment. In this way, the actuation mechanism 16 converts a manually exerted force into an actuation force acting on the mould members to bend the implant received between them. After the bending operation, the tabs 90, 92 are moved in the opposite direction (i.e., the surgical device 10 is "unfolded"), and the implant can be removed. The corresponding opening movement can be supported by a spring member comprised by the hinge mechanism 84, that urges the two support members 80, 82 in the non-actuated state illustrated in Fig. 6.

It should be noted that actuation mechanisms different from the mechanisms exemplarily illustrated in Figs. 1A, 1B, 5 and 6 could also be realized. For example, the mould members 12, 14 could be provided with multiple openings for being threaded onto multiple rods similar to the rod 62 of Fig. 5. By a relative movement of at least one of the mould members 12, 14 along the rods, the mould members 12, 14 could thus be actuated for bending purposes as described above.

As can be seen, the features described in the above description taken in conjunction with the accompanying drawings can be readily combined to result in different embodiments. It will thus be appreciated that the disclosure described above may be varied in many ways. Such variations are not to be regarded as a departure from the scope of the present invention, and all modifications are thus intended to be included within the scope of the claims that follow.

## Claims

1. An implant system comprising a surgical device (10) for bending an implant (60) and the implant (60) that has been bent by the surgical device (10), the device (10) comprising:
first and second mould members (12, 14) adapted to at least partially receive the implant (60) between them, the mould members (12, 14) being shaped in three dimensions in accordance with one or more cranio-facial features; and
an actuation mechanism (16) adapted to convert a manually exerted force into an actuation force acting on at least one of the mould members (12, 14) to bend the implant received between the mould members (12, 14);
**characterized in that**
the mould members (12, 14) are shaped in accordance with a portion of the orbita and are over-shaped relative to the bent implant (60) taking into account a partial elastic recovery of the implant (60) after bending so as to ensure that the shape of the elastically recovered implant exactly conforms to the one or more cranio-facial features, and **in that**
the implant system further comprises a fixation mechanism (56) defining a fixed relative position between the implant (60) and at least one of the mould members (14), wherein the fixation mechanism comprises two or more protrusions (56) provided on a face (52) of at least one of the mould members (14), the two or more protrusions (56) being adapted to be inserted into two or more corresponding openings (62) of the implant (60), said openings (62) adapted to receive bone screws for fixing the bent implant (60) to bone.

2. The implant system of claim 1, wherein the mould members (12, 14) are shaped in accordance with a generic model of the one or more cranio-facial features.

3. The implant system of any of claims 1 or 2, wherein the mould members (12, 14) have a patient-specific shape.

4. The implant system of any of the preceding claims, wherein the mould members (12, 14) are removably mounted to the surgical device.

5. The implant system of any of the preceding claims, wherein the fixation mechanism further comprises one or more abutment elements provided on a face of at least one of the mould members, the one or more abutment elements being adapted to be brought into abutment with portions of the implant (60).

6. The implant system of any of the preceding claims, wherein the first and second mould members (12, 14) are pivotally coupled to each other.

7. The implant system of claim 6, wherein the surgical device (10) is configured as pliers comprising two pivotally connected handles (22, 24) coupled to the first and second mould members (12, 14), respectively, wherein the handles (22, 24) are located on an opposite side of the first and second mould members with respect to the pivotal coupling and wherein the actuation mechanism (16) comprises the handles (22, 24).

8. The implant system of claim 6, wherein the surgical device (10) is configured as a handheld unit comprising a dedicated force exertion region (90, 92) associated with each of the first and second mould members (12, 14), that is located on the same side as the respective mould member (12, 14) with respect to the pivotal coupling, wherein the actuation mechanism (16) comprises the force exertion regions (90, 92).

9. The implant system of any of claims 1 to 5, wherein the surgical device (10) is configured as a tabletop unit comprising a base member supporting the first mould member (14) and a handle (34) coupled to the second mould member (12), wherein the actuation mechanism (16) comprises the handle (34).

10. The implant system of any of claims 1 to 9, wherein the implant (60) is an orbita implant.

11. The implant system of any of claims 1 to 10, wherein the implant (60) comprises a mesh structure.

12. The implant system of any of claims 1 to 11, wherein the implant (60) comprises one or more openings (62) adapted to receive bone screws.

13. The implant system according to any one of claims 1 to 12, comprising:
a first surgical device having a first pair of mould members shaped in accordance with a first anatomical side; and
a second surgical device having a second pair of mould members shaped in accordance with a second anatomical side opposite to the first anatomical side.

14. The implant system according to any one of claims 1 to 13, wherein the surgical device (10) is configured to removably receive the mould members (12, 14), and further comprising:
a first pair of mould members shaped in accordance with a first anatomical side; and
a second pair of mould members shaped in accordance with a second anatomical side opposite to the first anatomical side.

15. The implant system according to any one of claims 1 to 14, comprising:
a first implant shaped in accordance with a first anatomical side; and
a second implant shaped in accordance with a second anatomical side opposite to the first anatomical side.

## Patentansprüche

1. Implantatsystem, umfassend ein chirurgisches Gerät (10) zum Biegen eines Implantats (60) sowie das mittels des chirurgischen Geräts (10) gebogene Implantat (60), wobei das Gerät (10) umfasst:
erste und zweite Formteile (12, 14), die dazu ausgebildet sind, das Implantat (60) zumindest zum Teil zwischen sich aufzunehmen, wobei die Formteile (12, 14) in drei Dimensionen in Übereinstimmung mit einem oder mehreren Gesicht-Schädel-Merkmalen geformt sind; und
einen Betätigungsmechanismus (16), der dazu ausgebildet ist, eine manuell aufgebrachte Kraft in eine Betätigungskraft umzuwandeln, die auf mindestens eines der Formteile (12, 14) wirkt, um das zwischen den Formteilen (12, 14) aufgenommene Implantat zu biegen;
**dadurch gekennzeichnet, dass**
die Formteile (12, 14) in Übereinstimmung mit einem Teil der Orbita geformt sind und unter Berücksichtigung einer teilweisen elastischen Rückstellung des Implantats (60) nach dem Biegen gegenüber dem gebogenen Implantat (60) überformt sind, um sicherzustellen, dass die Gestalt des Implantats nach dessen elastischer Rückstellung exakt mit dem einen oder den mehreren Gesicht-Schädel-Merkmalen übereinstimmt, und dass
das Implantatsystem ferner einen Fixierungsmechnismus (56) umfasst, der eine feste Relativposition zwischen dem Implantat (60) und mindestens einem der Formteile (14) definiert, wobei der Fixierungsmechanismus zwei oder mehr an einer Oberfläche (52) mindestens eines der Formteile (14) vorgesehene Vorsprünge (56) umfasst, die dazu ausgebildet sind, in zwei oder mehr korrespondierende Öffnungen (62) des Implantats (60) eingesetzt zu werden, wobei die Öffnungen (62) dazu ausgebildet sind, Knochenschrauben zum Fixieren des gebogenen Implantats (60) am Knochen aufzunehmen.

2. Implantatsystem nach Anspruch 1, wobei die Formteile (12, 14) in Übereinstimmung mit einem generischen Model des einen oder der mehreren Gesicht-Schädel-Merkmale geformt sind.

3. Implantatsystem nach Anspruch 1 oder 2, wobei die Formteile (12, 14) eine patientenspezifische Gestalt haben.

4. Implantatsystem nach einem der vorherigen Ansprüche, wobei die Formteile (12, 14) lösbar an dem chirurgischen Gerät angebracht sind.

5. Implantatsystem nach einem der vorherigen Ansprüche, wobei der Fixierungsmechanismus ferner ein oder mehrere Anlageelemente umfasst, das/die auf einer Oberfläche mindestens eines der Formteile vorgesehen ist/sind und das/die dazu ausgebildet ist/sind, in Anlage mit Teilen des Implantats (60) gebracht zu werden.

6. Implantatsystem nach einem der vorherigen Ansprüche, wobei die ersten und zweiten Formteile (12, 14) schwenkbar miteinander gekoppelt sind.

7. Implantatsystem nach Anspruch 6, wobei das chirurgische Gerät (10) als Zange ausgebildet ist, die zwei schwenkbar verbundene Griffe (22, 24) umfasst, die jeweils mit den ersten und zweiten Formteilen (12, 14) gekoppelt und in Bezug auf die Schwenkkopplung an gegenüberliegenden Seiten der ersten und zweiten Formteile vorgesehen sind, und wobei der Betätigungsmechanismus (16) die Griffe (22, 24) umfasst.

8. Implantatsystem nach Anspruch 6, wobei das chirurgische Gerät (10) als Handgerät ausgebildet ist, das einen einem jeden der ersten und zweiten Formteile (12, 14) zugehörigen Bereich (90, 92) umfasst, der speziell für die Kraftaufbringung vorgesehen ist und in Bezug auf die Schwenkkopplung auf derselben Seite liegt wie das jeweilige Formteil (12, 14), und wobei der Betätigungsmechanismus (16) die Kraftaufbringungsbereiche (90, 92) umfasst.

9. Implantatsystem nach einem der Ansprüche 1 bis 5, wobei das chirurgische Gerät (10) als Tischgerät ausgebildet ist, das einen das erste Formteil (14) stützenden Basisteil sowie einen mit dem zweiten Formteil (12) gekoppelten Griff (34) umfasst, und wobei der Betätigungsmechanismus (16) den Griff (34) umfasst.

10. Implantatsystem nach einem der Ansprüche 1 bis 9, wobei das Implantat (60) ein Orbitaimplantat ist.

11. Implantatsystem nach einem der Ansprüche 1 bis 10, wobei das Implantat (60) eine Netzstruktur umfasst.

12. Implantatsystem nach einem der Ansprüche 1 bis 11, wobei das Implantat (60) eine oder mehrere Öffnungen (62) umfasst, die geeignet ist/sind, Knochenschrauben aufzunehmen.

13. Implantatsystem nach einem der Ansprüche 1 bis 12, umfassend:
ein erstes chirurgisches Gerät mit einem ersten Paar von in Übereinstimmung mit einer ersten anatomischen Seite geformten Formteilen; und
ein zweites chirurgisches Gerät mit einem zweiten Paar von in Übereinstimmung mit einer der ersten anatomischen Seite gegenüberliegenden, zweiten anatomischen Seite geformten Formteilen.

14. Implantatsystem nach einem der Ansprüche 1 bis 13, wobei das chirurgische Gerät (10) dazu ausgebildet ist, die Formteile (12, 14) lösbar aufzunehmen, und zudem umfasst:
ein erstes Paar von in Übereinstimmung mit einer ersten anatomischen Seite geformten Formteilen; und
ein zweites Paar von in Übereinstimmung mit einer der ersten anatomischen Seite gegenüberliegenden, zweiten anatomischen Seite geformten Formteilen.

15. Implantatsystem nach einem der Ansprüche 1 bis 14, umfassend:
ein in Übereinstimmung mit einer ersten anatomischen Seite geformtes erstes Implantat; und
ein in Übereinstimmung mit einer der ersten anatomischen Seite gegenüberliegenden, zweiten anatomischen Seite geformtes zweites Implantat.

## Revendications

1. Système d'implant comprenant un dispositif chirurgical (10) pour courber un implant (60) et l'implant (60) qui a été courbé par le dispositif chirurgical (10), le dispositif (10) comprenant :
des premier et deuxième éléments (12, 14) de moule adaptés à recevoir au moins partiellement l'implant (60) entre eux, les éléments (12, 14) de moule étant formés en trois dimensions en fonction d'une ou plusieurs caractéristique(s) craniofaciale(s) ; et
un mécanisme (16) d'actionnement adapté à convertir une force exercée manuellement en une force d'actionnement agissant sur au moins un des éléments (12, 14) de moule pour courber l'implant reçu entre les éléments (12, 14) de moule ;
**caractérisé en ce que**
les éléments (12, 14) de moule sont formés en fonction d'une partie de l'orbite et sont sur-formés par rapport à l'implant (60) courbé, prenant en compte une récupération élastique partielle de l'implant (60) après courbure de façon à assurer que la forme de l'implant ayant récupéré élastiquement se conforme exactement à la une ou aux plusieurs caractéristique(s) craniofaciale(s), et **en ce que**
le système d'implant comprend en outre un mécanisme (56) de fixation définissant une position relative fixe entre l'implant (60) et au moins un des éléments (14) de moule, dans lequel le mécanisme de fixation comprend deux ou plusieurs saillie(s) (56) prévue(s) sur une face (52) d'au moins un des éléments (14) de moule, les deux ou les plusieurs saillies (56) étant adaptées à être insérées dans deux ou plusieurs ouvertures (62) correspondantes de l'implant (60), lesdites ouvertures (62) adaptées à recevoir des vis pour os, afin de fixer l'implant (60) courbé à l'os.

2. Système d'implant selon la revendication 1, dans lequel les éléments (12, 14) de moule sont formés en fonction d'un modèle générique de la une ou des plusieurs caractéristique(s) craniofaciale(s).

3. Système d'implant selon l'une quelconque des revendications 1 ou 2, dans lequel les éléments (12, 14) de moule ont une forme spécifique au patient.

4. Système d'implant selon l'une quelconque des revendications précédentes, dans lequel les éléments (12, 14) de moule sont montés mobiles sur le dispositif chirurgical.

5. Système d'implant selon l'une quelconque des revendications précédentes, dans lequel le mécanisme de fixation comprend un ou plusieurs élément(s) de butée prévu(s) sur une face d'au moins un des éléments de moule, le un ou les plusieurs élément(s) de butée étant adapté(s) à être amené(s) en butée avec des parties de l'implant (60).

6. Système d'implant selon l'une quelconque des revendications précédentes, dans lequel les premier et deuxième éléments (12, 14) de moule sont couplés de façon pivotante l'un à l'autre.

7. Système d'implant selon la revendication 6, dans lequel le dispositif chirurgical (10) est configuré comme des pinces comprenant deux poignées (22, 24) connectées de façon pivotante couplées aux premier et deuxième éléments (12, 14) de moule, respectivement, dans lequel les poignées (22, 24) sont situées sur un côté opposé des premier et deuxième éléments de moule par rapport à l'accouplement pivotant et dans lequel le mécanisme (16) d'actionnement comprend les poignées (22, 24).

8. Système d'implant selon la revendication 6, dans lequel le dispositif chirurgical (10) est configuré comme une unité portable comprenant une région (90, 92) dédiée d'exercice de force associée avec chacun des premier et deuxième éléments (12, 14) de moule, qui est située sur le même côté que l'élément (12, 14) de moule respectif par rapport à l'accouplement pivotant, dans lequel le mécanisme (16) d'actionnement comprend les régions (90, 92) d'exercice de force.

9. Système d'implant selon l'une quelconque des revendications 1 à 5, dans lequel le dispositif chirurgical (10) est configuré comme une unité de table comprenant un élément de base supportant le premier élément (14) de moule et une poignée (34) couplée au deuxième élément (12) de moule, dans lequel le mécanisme (16) d'actionnement comprend la poignée (34).

10. Système d'implant selon l'une quelconque des revendications 1 à 9, dans lequel l'implant (60) est un implant pour orbite.

11. Système d'implant selon l'une quelconque des revendications 1 à 10, dans lequel l'implant (60) comprend une structure maillée.

12. Système d'implant selon l'une quelconque des revendications 1 à 11, dans lequel l'implant (60) comprend une ou plusieurs ouverture(s) (62) adaptée(s) à recevoir des vis pour os.

13. Système d'implant selon l'une quelconque des revendications 1 à 12, comprenant :
un premier dispositif chirurgical ayant une première paire d'éléments de moule formés en fonction d'un premier côté anatomique ; et
un deuxième dispositif chirurgical ayant une deuxième paire d'éléments de moule formés en fonction d'un deuxième côté anatomique opposé au premier côté anatomique.

14. Système d'implant selon l'une quelconque des revendications 1 à 13, dans lequel le dispositif chirurgical (10) est configuré pour recevoir de façon amovible les éléments (12, 14) de moule, et comprenant en outre :
une première paire d'éléments de moule formés en fonction d'un premier côté anatomique ; et
une deuxième paire d'éléments de moule formés en fonction d'un deuxième côté anatomique opposé au premier côté anatomique.

15. Système d'implant selon l'une quelconque des revendications 1 à 14, comprenant :
un premier implant formé en fonction d'un premier côté anatomique ; et
un deuxième implant formé en fonction d'un deuxième côté anatomique opposé au premier côté anatomique.
